# FASCICULE DE BREVET EUROPEEN

(11) **EP 1 317 911 B1**
(45) Date de publication et mention de la délivrance du brevet: **25.07.2007**
(21) Numéro de dépôt: 03005749.1
(22) Date de dépôt: 12.05.2000
(51) Int. Cl.: A61F 2/38

(54) **Prothèse de coude**
Ellbogenprothese
Elbow prosthesis

(30) Priorité: 14.05.1999 FR 9906314
(43) Date de publication de la demande: 11.06.2003
(62) Demande divisionnaire de: 00420094.5
(73) Titulaire: TORNIER SA, 38330 Saint Ismier (FR)
(72) Inventeur: Tornier, Alain, 38330 Saint Ismier (FR)
(74) Mandataire: Myon, Gérard Jean-Pierre

(56) Documents cités:
- EP-A- 0 006 314
- EP-A- 0 098 466
- EP-A- 0 913 133
- DE-C- 19 512 854
- FR-A- 2 663 838
- GB-A- 1 537 479
- US-A- 4 242 758
- US-A- 4 383 337
- US-A- 4 822 364
- US-A- 5 133 761

## Description

L'invention a trait à une prothèse de coude comprenant au moins un composant huméral et un composant cubital.

En fonction du jeu articulaire et du mode de couplage des éléments prothétiques huméral et cubital entre eux, on distingue principalement deux types de prothèses :
- les prothèses à charnière telles que connues du second mode de réalisation décrit dans EP-A-0 913 133 et dans lesquelles un axe d'articulation commun est introduit dans des orifices alignés prévus sur le composant huméral et sur le composant fémoral.
- les prothèses à glissement, telles que connues notamment de US-A-4,242,758 ou du premier mode de réalisation décrit dans EP-A-0 913 133, dans lesquelles le composant cubital vient en appui sur une surface d'articulation humérale globalement cylindrique.

L'invention vise à proposer une prothèse de coude qui autorise certains mouvements transversaux, tels que le mouvement de varus-valgus, tout en présentant une possibilité d'adaptation à la morphologie du patient.

Dans cet esprit, l'invention concerne une prothèse de coude comprenant un composant huméral formant une première surface d'articulation globalement cylindrique et un composant cubital formant une seconde surface d'articulation apte à être disposée autour d'une partie de cette première surface d'articulation et à pivoter autour d'un axe longitudinal de cette première surface d'articulation, caractérisée en ce qu'elle comprend des moyens d'ajustement, en fonction de la morphologie du patient, de la position de l'axe longitudinal de la première surface par rapport à l'axe longitudinal d'une tige d'ancrage du composant huméral.

En effet, la trochlée peut être plus ou moins en avant dans le plan sagittal par rapport à l'axe longitudinal de l'humérus, indépendamment de la taille de l'os, cette variation morphologique n'étant jusqu'à présent pas prise en compte par les prothèses de coude. La prothèse de la présente invention permet donc de tenir compte de cette variable lors de la pose d'une prothèse.

En particulier, on peut prévoir que la première surface est formée par une pièce allongée alors que des pattes solidaires de la tige d'ancrage sont pourvues de perçages de réception d'un axe de montage, la position relative des perçages de la pièce et des pattes étant adaptée en fonction de la morphologie du patient. Par exemple, la prothèse peut comprendre plusieurs pièces allongées et/ou plusieurs tiges, ces pièces et tiges étant aptes à être assemblées entre elles et permettant d'obtenir différentes positions de l'axe longitudinal des pièces globalement cylindriques par rapport aux axes des tiges. Selon une autre variante de réalisation, on peut prévoir que la tige et la pièce allongée formant la première surface sont fixes l'une par rapport à l'autre, notamment monobloc, la prothèse comprenant plusieurs ensembles de telles pièce et tige de géométries différentes pour une taille donnée d'humérus. Il est alors possible pour le chirurgien de choisir, lors de la pose de la prothèse, un ensemble tige-pièce en fonction de la morphologie du patient.

Selon d'autres aspects avantageux de l'invention :
- La première surface d'articulation a une génératrice concave, alors que la seconde surface d'articulation a, dans un plan de coupe parallèle à l'axe de la première surface d'articulation, une génératrice convexe. Grâce à cette disposition, l'axe du mouvement de varus-valgus est en permanence situé dans le composant cubital, ce qui donne une meilleure stabilité transversale à la prothèse ainsi réalisée, notamment, par rapport à celle connue de US-A-4,242,758 dans laquelle l'axe du mouvement de varus-valgus est, dans les configurations extrêmes, situé dans la bobine formant l'élément huméral.
- La première surface d'articulation se prolonge par une autre surface d'articulation, globalement convexe, d'appui d'un composant radial. Ceci permet de réaliser une prothèse totale de coude.
- Un jeu fonctionnel entre les surfaces précitées est formé par la différence de leurs diamètres ou des rayons de courbure de leurs génératrices respectives.
- Un orifice de passage d'au moins un fil de suture est ménagé à proximité de l'axe longitudinal de la première surface d'articulation et parallèlement à celui-ci.

L'invention sera mieux comprise et d'autres avantages de celle-ci apparaîtront plus clairement à la lumière de la description qui va suivre de deux modes de réalisation d'une prothèse de coude conforme à son principe, donnée uniquement à titre d'exemple et faite en référence aux dessins annexés dans lesquels :
- la figure 1 est une vue en perspective éclatée d'une prothèse de coude conforme à l'invention ;
- la figure 2 est une vue en perspective du composant cubital de la prothèse de la figure 1 en cours d'assemblage.
- La figure 3 est une vue de face de la prothèse de la figure 1 montée dans un coude en extension, dans une première configuration ;
- la figure 4 est une coupe selon le plan IV-IV à la figure 3 ;
- la figure 5 est une vue analogue à la figure 3 alors que la prothèse est dans une seconde configuration ;
- la figure 6 est une coupe selon le plan VI-VI à la figure 5 ;
- la figure 7 est une représentation schématique, à plus grande échelle et en coupe longitudinale, de certaines surfaces de contact de la prothèse des figures 1 à 6, et
- la figure 8 est une coupe partielle analogue à la figure 4 pour une prothèse conforme à un second mode de réalisation de l'invention.

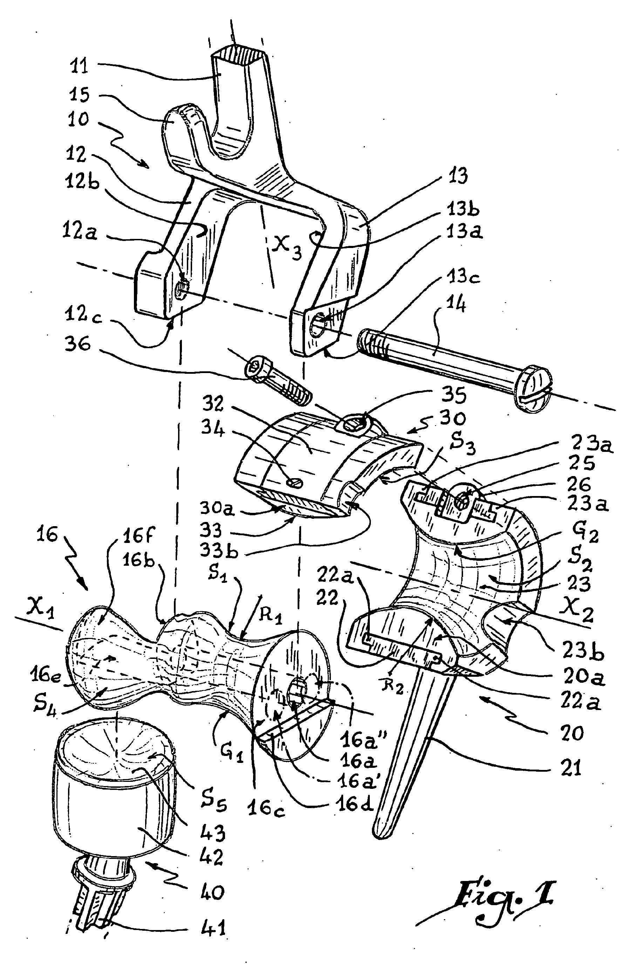

La prothèse visible aux figures 1 à 6 comprend un composant huméral 10 comportant une tige 11 destinée à être enfoncée dans le canal médullaire d'un humérus H et se prolongeant par deux pattes 12 et 13 percées chacune d'un orifice 12a ou 13a de passage d'une vis 14 formant axe. L'orifice 12a est taraudé, ce qui lui permet de coopérer avec le filetage extérieur de la vis 14.

La tige 11 se prolonge également par une troisième patte 15 destinée à venir en appui contre la corticale de l'humérus H et à prévenir une éventuelle bascule antérieure de la tige humérale.

Une pièce allongée 16 est pourvue d'un perçage central 16a dont les dimensions lui permettent de recevoir la vis 14. Deux surfaces d'extrémité 16b et 16c de la pièce 16 sont prévues pour venir respectivement au contact des surfaces en regard 12b et 13b des pattes 12 et 13, alors que la vis 14 traverse les orifices et perçages 13a, 16a et 12a.

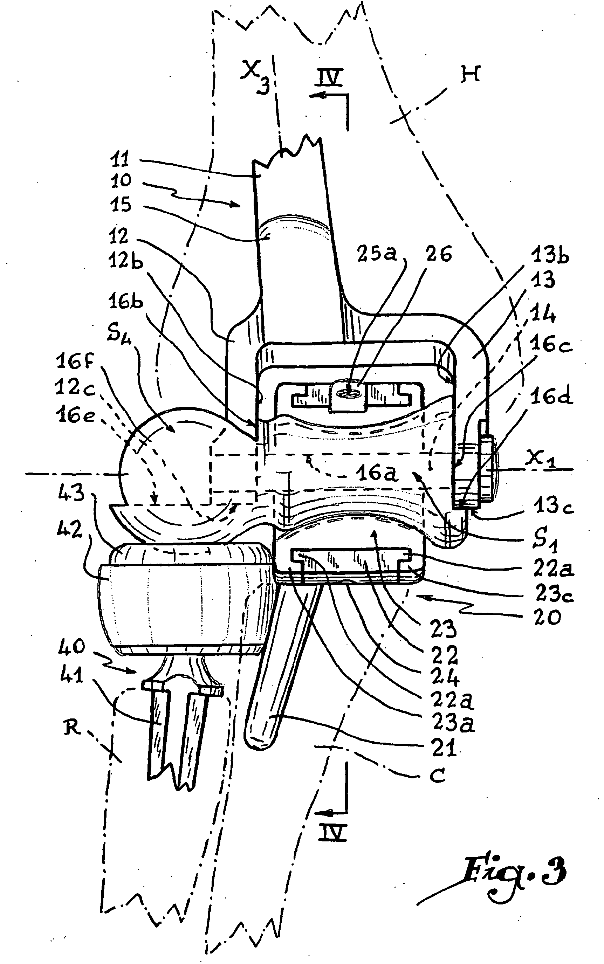

Dans cette position représentée aux figures 3 à 6, une surface d'extrémité 13c de la patte 13 est en appui contre un épaulement d'extrémité 16d de la pièce 16. De la même manière, une surface d'extrémité 12c de la patte 12 vient au contact d'une surface de forme complémentaire 16e prévue sur la pièce 16, au-delà de la surface 16b. Ainsi, la pièce 16 est immobilisée en rotation autour de la vis 14 par coopération de formes des surfaces 13c et 16d, d'une part, 12c et 16e, d'autre part. Entre les surfaces 16b et 16c, la pièce 16 est globalement cylindrique et forme une surface S₁ d'articulation également globalement cylindrique dont la génératrice G₁ est courbe et concave, en ce sens que le diamètre de la surface S₁ est minimal dans la partie centrale de la partie de la pièce 16 comprise entre les surfaces 16b et 16c. On note X₁ l'axe de symétrie de la surface S₁, D₁ son diamètre, qui est variable le long de l'axe X₁, et R₁ le rayon de courbure de la génératrice G₁ dans le plan de la figure 7.

La pièce 16 se prolonge par une extension 16f dont la surface externe S₄ est convexe. L'extension 16f coiffe la patte 12 lorsque la pièce 16 est montée sur les pattes 12 et 13.

L'ensemble du composant huméral 10 est métallique.

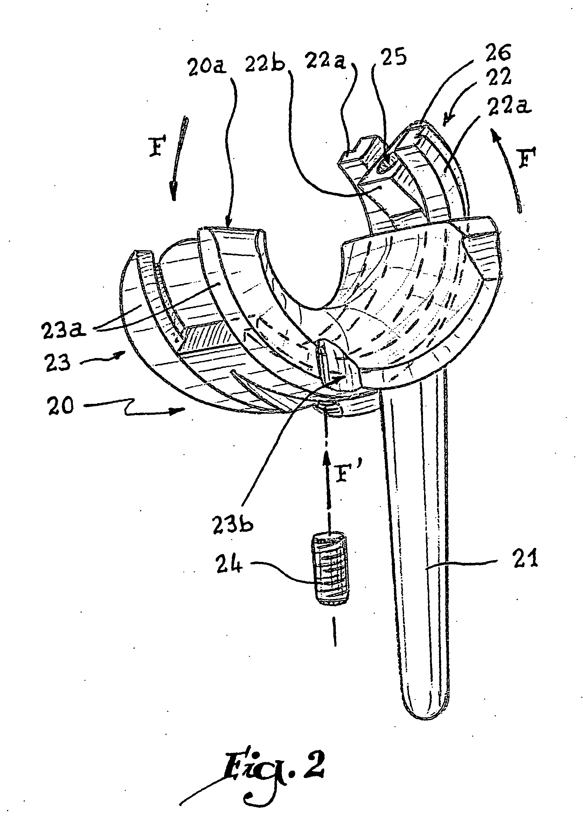

Un composant cubital 20 comprend une tige métallique 21 destinée à être insérée dans le canal médullaire du cubitus C et qui se prolonge par une patte 22 également métallique de forme concave dont la surface intérieure est revêtue d'une garniture 23 réalisée dans un matériau adapté au frottement avec la pièce métallique 16, par exemple en polyéthylène. La garniture 23 est montée sur la patte 22 par coopération de formes grâce à des retours 23a qui coiffent des ailettes latérales 22a de la patte 22. Une vis sans tête 24 permet d'immobiliser les éléments 22 et 23 l'un par rapport à l'autre. Le montage de la garniture 23 sur la patte 22 est représenté à la figure 2. La garniture 23 est engagée sur la patte 22 de telle sorte que ses retours 23a coiffent les ailettes 22a et subit un mouvement de glissement représenté par les flèches F jusqu'à venir en butée contre une surface d'arrêt 22b formée sur l'intérieur d'un bossage 26 de la patte 22. Lorsque la garniture 23 est en butée contre la surface 22b, la vis 24 est introduite et vissée dans la garniture 23 et dans la patte 22, comme représenté par la flèche F'. La patte 22 constitue alors une armature enveloppante pour la garniture 23.

Le procédé d'assemblage utilisé entre la garniture 23, qui peut notamment être réalisé en matière plastique, et la patte 22, qui est métallique, présente les avantages suivants :
- une grande facilité d'usinage de la garniture 23 car sa matière constitutive peut être usinée uniquement par tournage, sans avoir recours à des machines à commande numérique.
- un maintien particulièrement efficace de la garniture 23 sur la patte 22.
- la présence d'une quantité importante de matière, par exemple de polyéthylène, formant la garniture 23 de part et d'autre du métal formant la patte 22, ce qui permet d'éviter tout contact entre les parties métalliques 16 et 22 lors du mouvement articulaire.

Bien que le mode d'assemblage décrit ci-dessus soit particulièrement avantageux, l'immobilisation relative des éléments 22 et 23 peut être effectuée par d'autres moyens, notamment par blocage, sertissage ou clipsage.

La garniture 23 est pourvue d'une encoche latérale 23b afin de ne pas interférer avec les tissus passant à proximité du composant 20 en position montée de la prothèse.

La surface interne de la garniture 23 forme une surface d'articulation S₂ de forme complémentaire de la surface S₁. On note X₂ l'axe central des éléments 22 et 23, D₂ le diamètre de la surface S₂, ce diamètre étant variable le long de l'axe X₂. En coupe dans un plan parallèle à l'axe X₂, la surface S₂ est convexe en ce sens que sa génératrice G₂ est courbe et convexe, avec un centre de courbure disposé du côté de la patte 22, c'est-à-dire l'opposé de l'axe X₂. On note R₂ le rayon de courbure de la génératrice G₂.

Les diamètres respectifs D₁ et D₂ et les rayons de courbure respectifs R₁ et R₂ des surfaces S₁ et S₂ sont sensiblement égaux. Les légères différences de valeurs de D₁ et D₂ d'une part et de R₁ et R₂ d'autre part permettent de créer un jeu fonctionnel diamétral J et un jeu fonctionnel longitudinal J' qui sont exagérés sur la figure 7 pour la clarté du dessin. Ces jeux fonctionnels J et J' qui ont des valeurs faibles, permettent le mouvement relatif de varus-valgus et en rotation axiale du composant cubital 20 par rapport au composant huméral 10.

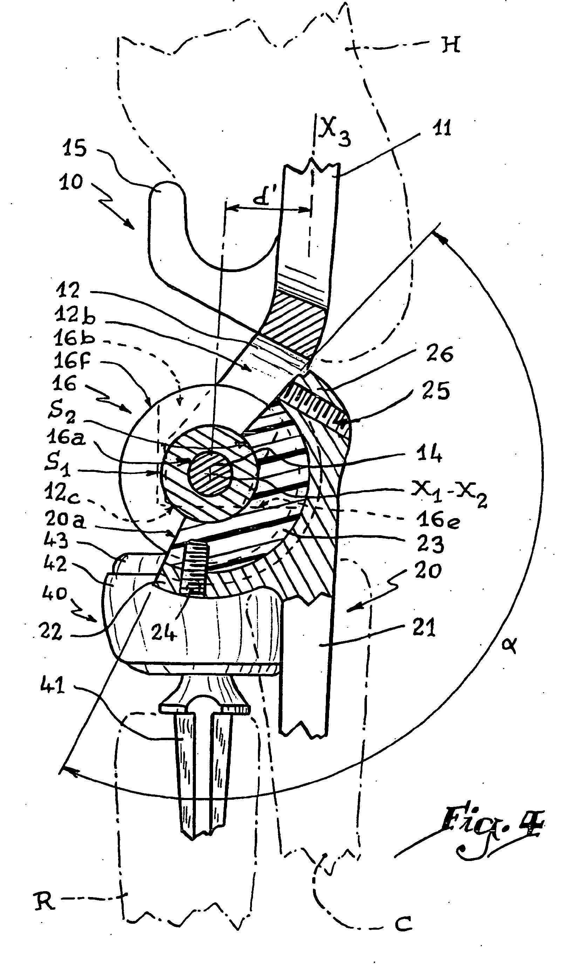

Les dimensions et orientations respectives des surfaces S₁ et S₂ sont telles que le composant cubital peut être disposé autour d'une partie de la surface S₁, comme représenté aux figure 3 et 4. Dans cette position, la garniture 23 entoure la pièce 16 sur une partie de sa circonférence représentée à la figure 4 par un angle α définissant la portée de la congruence entre les surfaces S₁ et S₂, visible à la figure 4 et inférieur à environ 180°. L'angle α est inférieur à 180° pour permettre la mise en place du composant cubital sur la pièce 16. Les jeux de fonctionnement J et J' prévus entre les surfaces S₁ et S₂ peuvent permettre que l'angle α soit supérieur à 180°, sans compromettre le libre montage du composant 20 sur la pièce 16.

Dans cette configuration, la prothèse se comporte comme une prothèse à glissement.

Lorsque le chirurgien réalise que les tendons et ligaments de l'articulation sont en bon état et qu'il y a peu de risques que l'articulation puisse se luxer, la prothèse peut être utilisée dans la configuration des figures 3 et 4.

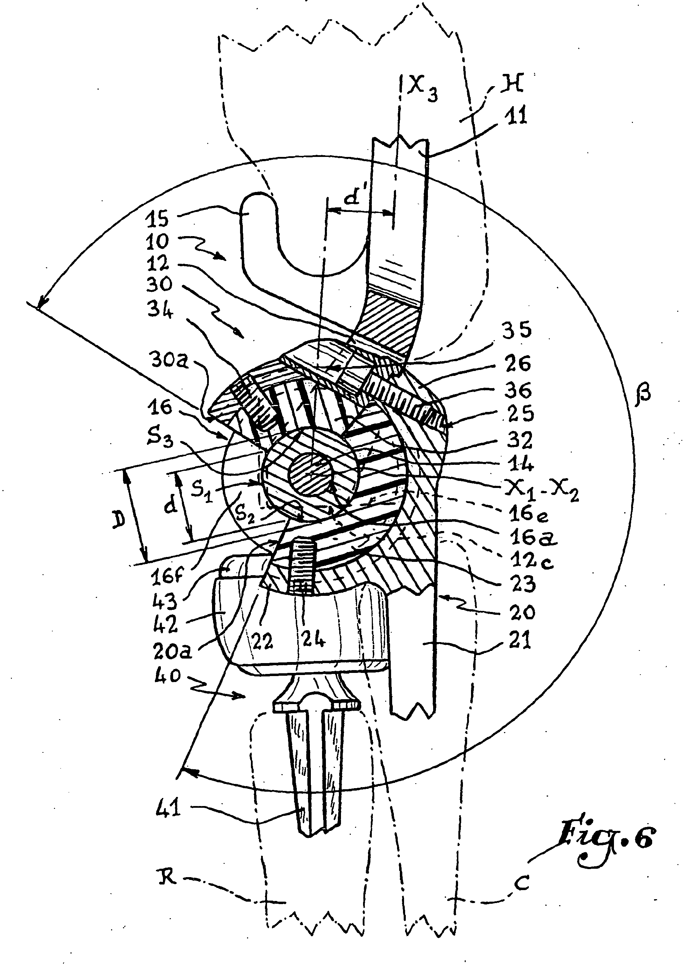

Une sécurité accrue de l'articulation prothétique peut être obtenue en utilisant un élément de verrouillage 30 destiné à être monté sur le composant cubital 20 et formé d'une armature 32 et d'une garniture 33 immobilisées l'une par rapport à l'autre grâce à des ailettes et retours analogues à ceux de la patte 22 et de la garniture 23 et à une vis sans tête 34 visible à la figure 6. La garniture 33 est réalisée dans un matériau adapté au frottement avec la pièce 16, par exemple en polyéthylène, ce matériau étant avantageusement identique à celui de la garniture 23. Le montage de la garniture 33 sur l'armature 32 a lieu d'une façon analogue à celle décrite en référence à la figure 2.

Une encoche latérale 33b est prévue sur la garniture 33, cette encoche étant diamétralement opposée à l'encoche 23b en position montée de l'élément 30 et ayant la même fonction que l'encoche 23b.

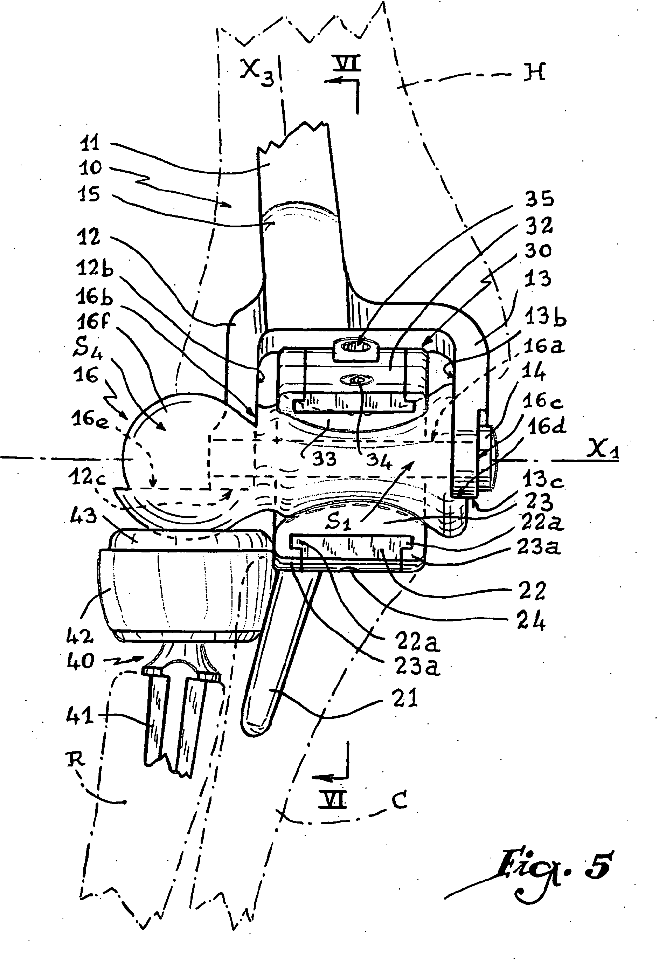

La surface interne de la garniture 33 forme une troisième surface d'articulation S₃ de géométrie comparable à celle de la surface S₂. Plus précisément, les génératrices des surfaces S₂ et S₃ sont sensiblement identiques, de telle sorte que lorsque l'élément 30 est monté sur le composant 20, la surface S₃ prolonge la surface S₂. On est alors dans la configuration des figures 5 et 6 dans laquelle les surfaces S₂ et S₃ de l'ensemble formé des éléments 20 et 30 enserrent la surface S₁, les axes X₁ et X₂ pouvant se déplacer l'un par rapport à l'autre, tant en translation qu'en rotation, de distances ou d'angles donnés par la valeur des jeux fonctionnels diamétral et longitudinal J et J' ménagés entre la surface S₁ et la combinaison des surfaces S₂ et S₃. En effet, la combinaison des surfaces S₁ et S₂ et S₃ permet d'obtenir :
- une surface de contact suffisante entre les pièces 16 et 20 ou 16, 20 et 30, quelle que soit la position relative des composants huméral et cubital.
- la possibilité d'un mouvement de varus-valgus et d'une rotation axiale, quels que soient les efforts subis par ces composants.
- la possibilité d'une translation médio-latérale du cubitus sur l'humérus.
- la possibilité d'une translation antéropostérieure du cubitus sur l'humérus.

Dans le plan de la figure 6, les surfaces S₂ et S₃ entourent ensemble la surface S₁ sur un angle β de l'ordre de 270°. En fait, le verrouillage obtenu grâce à l'élément 30 est efficace pour autant que l'angle β est supérieur à 180°. Des résultats satisfaisants ont été obtenus avec un angle β compris entre 225 et 315°, cet angle pouvant raisonnablement être étendu sur une plage allant de 190 à 360°.

Pour autant que l'angle β est supérieur à 180°, la coopération des surfaces S₁ d'une part, S₂ et S₃ d'autre part, permet d'éviter un déboîtement ou une luxation de l'articulation, alors que des mouvements de varus-valgus demeurent possibles par glissement transversal et/ou pivotement des surfaces S₂ et S₃ par rapport à la surface S₁.

Le montage de l'élément 30 sur le composant 20 est obtenu grâce à un taraudage 25 pratiqué dans le bossage 26 prévu sur la patte 22, alors qu'un logement 35 est prévu dans l'armature 32 pour la réception d'une vis 36 destinée à être serrée dans le taraudage 25. Selon une solution miroir, un taraudage peut être prévu dans l'armature 32 alors qu'un logement réception d'une tête de vis est prévu dans la patte 22. D'autres moyens de fixation peuvent être envisagés, notamment des pions, éventuellement tronconiques, ou un assemblage par tenons et mortaises.

Dans la configuration des figures 5 et 6, la distance d, entre les bords libres respectifs 20a et 30a du composant 20 et de l'élément 30, est inférieure au diamètre minimum D de la surface S₁, ce qui correspond au verrouillage recherché grâce à l'élément 30.

On note X₃ l'axe longitudinal de la tige 11 et d' le décalage des axes X₁ et X₃ dans le plan des figures 4 et 6. La valeur de d' est un paramètre dépendant de la morphologie du patient.

Il est possible d'ajuster la valeur de d' en utilisant différentes pièces 16 dont le perçage central 16a est plus ou moins décalé par rapport à l'axe X₁, comme représenté en traits mixtes à la figure 1 avec les références 16a' et 16a''. Ainsi, après essai de la tige 11 dans l'humérus, le chirurgien peut choisir, parmi plusieurs pièces 16 dont le perçage central est plus ou moins décalé par rapport à l'axe X₁, celle qui correspond le mieux à la position anatomique de la trochlée.

Dans tous les cas, les surfaces 12c et 13c des pattes 12 viennent respectivement en appui contre les surfaces 16d et 16e de la pièce 16.

D'autres variantes permettent d'obtenir le même résultat, notamment l'utilisation de pièces formant les parties 11 à 15 de géométrie variable, les orifices 12a et 13a étant plus ou moins décalés par rapport à l'axe X₃. Dans ce cas, une unique pièce allongée 16 peut être utilisée.

Selon une autre alternative, les éléments 11 à 16 peuvent être formés de façon monobloc, un ensemble formant prothèse comprenant différents ensembles monobloc dont le décalage d' est variable. Le chirurgien peut ainsi choisir, parmi ces ensembles monobloc, la pièce la mieux adaptée après mise en place provisoire d'une prothèse fantôme et essai.

En pratique, quel que soit le mode de réglage du décalage envisagé, il apparaît qu'une amplitude de plus ou moins 5 mm autour de la position médiane représentée en traits pleins à la figure 1 permet de couvrir la plupart des cas opératoires.

Un composant radial 40 est prévu et comprend une tige 41 destinée à être insérée dans le canal médullaire du radius R et une tête 43 en matière plastique, par exemple en polyéthylène ceinturée par une frette 42 métallique. La tête 43 forme une surface d'articulation concave S₅ prévue pour venir en appui contre la surface S₄ de la pièce 16. Dans cette configuration la prothèse de l'invention est une prothèse totale.

Selon une variante non représentée de l'invention, la prothèse peut être partielle en ce sens qu'elle ne comprend pas de composant radial, la tête anatomique du radius étant alors directement articulée sur la surface métallique S₄.

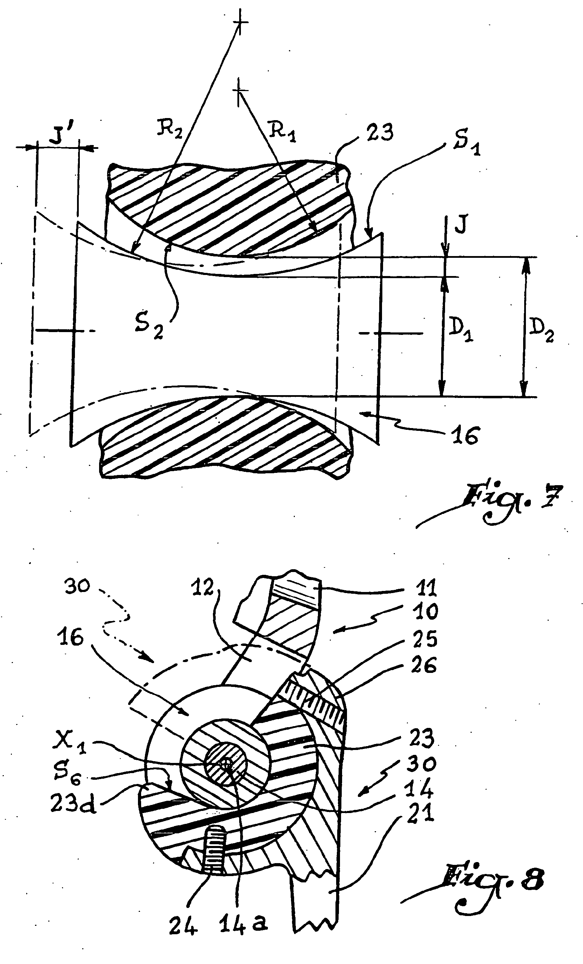

Dans le second mode de réalisation de l'invention représenté à la figure 8, les éléments analogues à ceux du premier mode de réalisation portent des références identiques. Un sabot 23d prolonge la garniture 23 à l'opposé de son bord prévu pour entrer en contact avec l'élément 30. La surface interne S₆ du sabot 23d prolonge tangentiellement la surface S₂, de telle sorte qu'elle constitue une protection supplémentaire contre une luxation de la prothèse, utilisable avec ou sans l'élément 30 qui peut être monté sur le composant 20 dans la position représentée en traits mixtes. Le verrouillage obtenu est encore amélioré par rapport au premier mode de réalisation.

Selon un aspect avantageux mais non obligatoire de l'invention, un orifice axial 14a est pratiqué longitudinalement dans la vis 14 de façon à permettre le passage de fils de suture destinés à attacher les structures ligamentaires au contact de la prothèse ou de l'os, à proximité de leur point d'ancrage anatomique situé sur l'axe de flexion du coude. Un orifice équivalent peut également être pratiqué dans la pièce 16, à proximité de l'axe X₁ et parallèlement à celui-ci, notamment dans la variante monobloc évoquée ci-dessus.

Selon une variante de l'invention applicable quel que soit le mode de réalisation considéré, le jeu entre les surfaces S₁ et S₂ ou entre la surface S₁ et la combinaison des surfaces S₂ et S₃ peut être uniquement diamétral ou, au contraire, ne pas comprendre de jeu diamétral ou avoir un jeu diamétral très faible, le jeu fonctionnel étant alors essentiellement longitudinal. Dans le premier cas, une bonne résistance aux mouvements de varus-valgus et de rotation est obtenue, au détriment de la mobilité transversale. Dans le second cas, le fonctionnement est correct, mais une certaine usure des surfaces S₁, S₂ et S₃ est prévisible.

## Revendications

1. Prothèse de coude comprenant un composant huméral (10) formant une première surface d'articulation (S₁) globalement cylindrique et un composant cubital (20) formant une seconde surface d'articulation (S₂) apte à être disposée autour d'une partie de ladite première surface d'articulation et à pivoter autour d'un axe longitudinal (X₁) de ladite première surface d'articulation, **caractérisée en ce qu'**elle comprend des moyens (12a, 13a, 16a; 11, 16, 16a, 16a', 16a") d'ajustement, en fonction de la morphologie du patient, de la position (d') de l'axe longitudinal (X₁) de ladite première surface (S₁) par rapport à l'axe longitudinal (X₃) d'une tige (11) d'ancrage dudit composant huméral (10).

2. Prothèse selon la revendication 1, **caractérisée en ce que** ladite première surface (S₁) est formée par une pièce allongée (16) alors que des pattes (12, 13) solidaires de la tige sont pourvues de perçages (12a, 13a, 16a) de réception d'un axe de montage (14) de ladite pièce, la position relative desdits perçages de ladite pièce et desdites pattes étant adaptée en fonction de la morphologie du patient.

3. Prothèse selon l'une des revendications 1 ou 2, **caractérisée en ce que** ladite première surface (S₁) est formée dans une pièce allongée alors que la prothèse comprend plusieurs pièces allongées (16) et/ou plusieurs tiges (11) d'ancrage lesdites pièces et tiges étant aptes à être assemblées entre elles et permettant d'obtenir différentes positions (d') dudit axe longitudinal (X₁) desdites pièces par rapport aux axes (X₃) desdites tiges.

4. Prothèse selon la revendication 3, **caractérisée en ce que** lesdites pièces allongées (16) ont des perçages centraux (16a, 16a', 16a") de réception d'une vis (14) de montage sur ledit composant (10-13) qui sont plus ou moins décalés par rapport audit axe longitudinal (X₁) de ladite première surface (S₁).

5. Prothèse selon la revendication 2, **caractérisée en ce que** ladite tige (11) et une pièce allongée (16) formant ladite première surface (S₁) sont fixes l'une par rapport à l'autre, notamment monobloc, ladite prothèse comprenant plusieurs ensembles de pièces et tiges de géométries différentes.

6. Prothèse selon l'une des revendications précédentes, **caractérisée en ce que** ladite première surface d'articulation (S₁) a une génératrice (G₁) concave, alors que ladite seconde surface d'articulation (S₂) a, dans un plan de coupe parallèle à l'axe (X₁) de ladite première surface d'articulation, une génératrice convexe (G₂).

7. Prothèse selon l'une des revendications précédentes, **caractérisée en ce que** ladite première surface d'articulation (S₁) se prolonge par une autre surface d'articulation (S₄), globalement convexe, d'appui d'un composant radial (40).

8. Prothèse selon l'une des revendications précédentes, **caractérisé en ce qu**'un jeu fonctionnel (J, J') entre lesdites surfaces (S₁, S₂) est formé par la différence des diamètres (D₁, D₂) desdites surfaces et/ou des rayons de courbure (R₁, R₂) de leurs génératrices respectives (G₁, G₂).

9. Prothèse selon l'une des revendications précédentes, **caractérisée en ce qu**'elle comprend un orifice (14a) de passage d'au moins un fil de suture ménagé à proximité dudit axe longitudinal (X₁) de ladite première surface d'articulation (S₁) et parallèlement à celui-ci.

## Claims

1. An elbow prosthesis comprising a humeral component (10) forming a first, on the whole cylindrical joint surface (S₁) and a cubital component (20) forming a second joint surface (S₂) capable of being disposed around part of said first joint surface and of pivoting around a longitudinal axis (X₁) of said first joint surface, **characterised in that** it comprises means (12a, 13a, 16a; 11, 16, 16a, 16a', 16a") of adjusting, as a function of the morphology of the patient, the position (d') of the longitudinal axis (X₁) of said first surface (S₁) relative to the longitudinal axis (X₃) of an anchoring rod (11) of said humeral component (10).

2. A prosthesis according to claim 1, **characterised in that** said first surface (S₁) is formed by an elongate element (16) while lugs (12, 13) integral with the rod are provided with bores (12a, 13a, 16a) for receiving a mounting shaft (14) of said element, the relative position of said bores of said element and said lugs being adjusted as a function of the morphology of the patient.

3. A prosthesis according to one of claims 1 or 2, **characterised in that** said first surface (S₁) is formed in an elongate element while the prosthesis comprises a plurality of elongate elements (16) and/or a plurality of anchoring rods (11), said elements and rods being capable of being fitted together and allowing the obtainment of various positions (d') of said longitudinal axis (X₁) of said elements relative to the axes (X₃) of said rods.

4. A prosthesis according to claim 3, **characterised in that** said elongate elements (16) have central bores (16a, 16a', 16a'') for receiving a screw (14) for mounting them on said component (10-13), said bores (16a, 16a', 16a'') being offset to a greater or lesser degree relative to said longitudinal axis (X₁) of said first surface (S₁).

5. A prosthesis according to claim 2, **characterised in that** said rod (11) and an elongate element (16) forming said first surface (S₁) are fixed relative to one another, in particular are in one piece, said prosthesis comprising a plurality of assemblies of elements and rods of different geometries.

6. A prosthesis according to one of the preceding claims, **characterised in that** said first joint surface (S₁) has a concave generating line (G₁), while said second joint surface (S₂) has, in a section plane parallel to the axis (X₁) of said first joint surface, a convex generating line (G₂).

7. A prosthesis according to one of the preceding claims, **characterised in that** said first joint surface (S₁) is extended by another, on the whole convex, joint surface (S₄), providing support for a radial component (40).

8. A prosthesis according to one of the preceding claims, **characterised in that** functional play (J, J') between said surfaces (S₁, S₂) is formed by the difference in the diameters (D₁, D₂) of said surfaces and/or in the radii of curvature (R₁, R₂) of their respective generating lines (G₁, G₂).

9. A prosthesis according to one of the preceding claims, **characterised in that** it comprises an orifice (14a) for passage of at least one suture thread, formed in the vicinity of said longitudinal axis (X₁) of said first joint surface (S₁) and parallel thereto.

## Patentansprüche

1. Ellenbogenprothese, umfassend eine Humeruskomponente (10), die eine im Wesentlichen zylindrische erste Artikulationsoberfläche (S₁) bildet, und eine Cubituskomponente (20), die eine zweite Artikulations-oberfläche (S₂) bildet und derart ausgelegt ist, dass sie um einen Teil der ersten Artikulationsoberfläche herum angebracht werden und um eine Längsachse (X₁) der ersten Artikulationsoberfläche herum schwenken kann, **dadurch gekennzeichnet, dass** sie Mittel (12a, 13a, 16a; 11, 16, 16a, 16a', 16a") zum Einstellen der Position (d') der Längsachse (X₁) der ersten Oberfläche (S₁) in Bezug auf die Längsachse (X₃) eines Stifts (11) zur Verankerung der Humeruskomponente (10) je nach der Morphologie des Patienten aufweist.

2. Prothese nach Anspruch 1, **dadurch gekennzeichnet, dass** die erste Oberfläche (S₁) durch ein längliches Teil (16) gebildet wird, während mit dem Stift einstückige Laschen (12, 13) mit Bohrungen (12a, 13a, 16a) zum Aufnehmen einer Montageachse (14) des Teils versehen sind, wobei die relative Position dieser Bohrungen des Teils und der Laschen je nach der Morphologie des Patienten angepasst wird.

3. Prothese nach einem der Ansprüche 1 oder 2, **dadurch gekennzeichnet, dass** die erste Oberfläche (S₁) in einem länglichen Teil gebildet ist, während die Prothese mehrere längliche Teile (16) und/oder mehrere Verankerungsstifte (11) umfasst, wobei diese Teile und Stifte untereinander zusammengebaut werden können und es ermöglichen, verschiedene Positionen (d') der Längsachse (X₁) der Teile in Bezug auf die Achsen (X₃) der Stifte zu erhalten.

4. Prothese nach Anspruch 3, **dadurch gekennzeichnet, dass** die länglichen Teile (16) zentrale Bohrungen (16a, 16a', 16a") zum Aufnehmen eines Gewindes (14) für die Montage an der Komponente (10-13) besitzen, die in Bezug auf die Längsachse (X₁) der ersten Oberfläche (S₁) mehr oder weniger verschoben sind.

5. Prothese nach Anspruch 2, **dadurch gekennzeichnet, dass** der Stift (11) und ein längliches Teil (16), das die erste Oberfläche (S₁) bildet, aneinander befestigt und insbesondere ein Monoblock sind, wobei die Prothese mehrere Gruppen von Teilen und Stiften mit unterschiedlichen Geometrien umfasst.

6. Prothese nach einem der vorhergehenden Ansprüche, **dadurch gekennzeichnet, dass** die erste Artikulations-oberfläche (S₁) eine konkave Mantellinie (G₁) besitzt, während die zweite Artikulationsoberfläche (S₂) in einer Schnittebene parallel zur Achse (X₁) dieser ersten Artikulationsoberfläche eine konvexe Mantellinie (G₂) aufweist.

7. Prothese nach einem der vorhergehenden Ansprüche, **dadurch gekennzeichnet, dass** die erste Artikulations-oberfläche (S₁) sich in einer anderen, im Wesentlichen konvexen Artikulationsoberfläche (S₄) zur Auflage einer radialen Komponente (40) erstreckt.

8. Prothese nach einem der vorhergehenden Ansprüche, **dadurch gekennzeichnet, dass** ein Spiel (J, J') zwischen den Oberflächen (S₁, S₂) durch den Unterschied der Durchmesser (D₁, D₂) dieser Oberflächen und/oder die Krümmungsradien (R₁, R₂) ihrer jeweiligen Mantellinien (G₁, G₂) gebildet wird.

9. Prothese nach einem der vorhergehenden Ansprüche, **dadurch gekennzeichnet, dass** sie eine Öffnung (14a) für das Hindurchtreten mindestens eines Nahtfadens umfasst, die in der Nähe der Längsachse (X₁) der ersten Artikulationsoberfläche (S₁) und parallel zu dieser hergestellt wurde.
